# EUROPEAN PATENT APPLICATION

(11) **EP 3 662 900 A1**
(43) Date of publication of application: **10.06.2020**
(21) Application number: 18211141.9
(22) Date of filing: 07.12.2018
(51) Int. Cl.: A61K 9/28, A61K 31/606

(54) **COLONIC DRUG DELIVERY FORMULATION**

(71) Applicant: Tillotts Pharma AG, 4310 Rheinfelden (CH)
(72) Inventor: VARUM, Felipe, 4310 Rheinfelden (CH); BRAVO GONZALEZ, Roberto Carlos, 4310 Rheinfelden (CH)
(74) Representative: Beck Greener LLP

(57) **Abstract**

In a delayed release drug formulation comprising a core containing a drug and optionally and a delayed release coating for intestinal release, release of the drug in the colon is not hindered by the absence of an alkaline middle layer between the core and the outer layer. The delayed release coating comprising and inner coating and optionally an isolation layer. The outer coating comprises a mixture of an enzymatically degradable polysaccharide which is degradable by colonic enzymes selected from the group consisting of starch, amylose, amylopectin, chitosan, chondroitin sulfate, cyclodextrin, dextran, pullulan, carrageenan, scleroglucan, chitin, curdulan, and levan; and a film-forming enteric polymer having a pH threshold at about pH 6 or above, wherein the enzymatically degradable polysaccharide and the enteric polymer are present in the outer coating in a ratio of more than 60:40.

## Description

The present invention relates to a delayed release drug formulation for oral administration with a core comprising a drug, and a delayed release coating for the core. In particular, it relates to a delayed release formulation for a drug for delivering to the colon.

The targeting of drugs to the intestine is well known and has been known for over one hundred years. Commonly, the target of the drugs is the small intestine although the colon can be utilised as a means of achieving local therapy or systemic treatment. The requirements for the coatings on the drugs are different depending on the target site. In order to reach the colon, it is necessary for the drugs to pass through the small intestine, and therefore it is a requirement that a delayed release coating intended to release the drug in the colon does not release the drug in the small intestine.

Coated products for release in the small intestine commonly use polymer coatings which dissolve or disintegrate in a pH dependent manner. In the low pH environment of the stomach, the polymer coating is insoluble. However, on reaching the small intestine, the pH rises to 5 and above and the polymeric coating dissolves or disintegrates. A commonly used coating is one containing ionizable carboxylic groups. At higher pH levels, the carboxylic groups ionize, allowing the polymer coatings to disintegrate or dissolve. Common polymers of this type which are used include Eudragit® L and Eudragit® S.

Various methods of improving the release in the small intestine by ensuring an earlier release of the drug are known. US2008/0200482 is one of a number of references which discloses partially neutralizing the carboxylic groups in order to reduce the pH at which disintegration occurs. WO2008/135090 discloses a tablet with an inner coat of partially neutralized material and an outer coat with less or no neutralization. This is said to result in disintegration at an earlier time point when transferred from the stomach.

Release of drugs in the colon typically requires an alternative approach. The colon is susceptible to a number of disease states, including inflammatory bowel disease, irritable bowel syndrome, constipation, diarrhoea, infection and carcinoma. In such conditions, drug targeting to the colon would maximise the therapeutic effectiveness of the treatment. The colon can also be utilised as a portal for the entry of drugs into the systemic circulation. Various formulations have been developed for colonic drug delivery, including pro-drugs as well as formulated dosage forms, with the latter being more popular since the concept once proved can be applied to other drugs.

The high bacterial population of the colon has also been exploited in developing colonic drug delivery dosage forms through the use, as digestible carrier materials, of naturally occurring polysaccharides that constitute substrates for the numerous enzymes produced by the resident colonic bacteria. These materials are able to pass through the upper gastrointestinal regions intact but are digested upon entry into the colon. Examples include starch, amylose, amylopectin, pectin, chitosan, galactomannan and guar gum.

One major attraction of using polysaccharides in this bacterial enzyme approach to colonic drug delivery is that materials used are of at least food grade and so would be safe for use in humans. They are usually applied as coatings or incorporated in the core material as a matrix carrier, and their digestion on entry into the colon by the colonic bacterial enzymes leads to the release of the drug load. An example of such a formulation, which employs an amylose coating, is disclosed in EP0343993A (BTG International Limited).

A major limitation with these naturally occurring materials, however, is that they swell excessively in aqueous media leading to leaching of the drug load in the upper gastrointestinal regions. To circumvent this problem, the naturally occurring materials have been utilised in a mixture with various water-insoluble materials.

EP0502032A (British Technology Group Ltd) teaches the use of an outer coating comprising a film-forming cellulose or an acrylate polymer material and amorphous amylose for a tablet comprising an active compound. The polymer material used is a pH independent release polymer material.

An article in Journal of Controlled Release (Milojevic et al; 38; (1996); 75-84) reports the results of investigations concerning the incorporation of a range of insoluble polymers into an amylose coating in order to control amylose swelling. A range of cellulose and acrylate based co-polymers are assessed, and a commercially available ethyl cellulose (Ethocel®) is found to control the swelling most effectively. A pH dependent soluble coating of Eudragit® L100 is employed but only in a multi-layer system comprising a bioactive coated with an inner coating of amylose and then an outer coating of Eudragit® L 100.

A further amylose-based coating composition is disclosed in WO99/21536A (BTG International Limited). The coating composition comprises a mixture of amylose and a water insoluble pH independent film-forming polymer which is formed from a water-insoluble cellulosic or acrylate polymer material.

WO99/25325A (BTG International Limited) also discloses a delayed release coating comprising amylose and (preferably) ethyl cellulose or alternatively an insoluble acrylate polymer. The coating composition also includes a plasticiser and the method finds particular application in the preparation of dosage forms comprising active materials that are unstable at temperatures in excess of 60°C, as the composition is formed at lower temperatures than this.

WO03/068196A (Alizyme Therapeutics Ltd) discloses a specific delayed release coating for the bioactive prednisolone sodium metasulphobenzoate comprising glassy amylose, ethyl cellulose and dibutyl sebacate.

The use of polysaccharides other than amorphous amylose in a delayed release coating is disclosed in GB2367002 (British Sugar PLC). Examples include guar gum, karaya gum, gum tragacanth and xanthan gum. Microparticles of these polysaccharides are dispersed in a water-insoluble film-forming polymer matrix formed for example from a cellulose derivative, an acrylic polymer or a lignin.

WO01/76562A (Tampereen Patenttitoimisto Oy) discloses a peroral pharmaceutical formulation containing a drug and a chitosan (a polysaccharide obtained from chitin) for controlling its release. The drug and the chitosan are mixed into a homogeneous mechanical powder mixture which is granulated and then optionally tableted. The granulation may be performed with an enteric polymer (such as a copolymer of methacrylic acid) or the granules may be provided with a porous enteric coating.

WO2004/052339A (Salvona LLC) discloses a pH dependent drug release system which is a free-flowing powder of solid hydrophobic nano-spheres comprising a drug encapsulated in a pH-sensitive micro-sphere. The nano-spheres are formed from the drug in combination with a wax material, and the pH-sensitive micro-sphere formed from a pH-sensitive polymer (such as a Eudragit® polymer) in combination with a water-sensitive material such as a polysaccharide.

An article in the European Journal of Pharmaceutical Sciences (Akhgari et al; 28; March 2006; 307-314) reports the results of investigations into the use of certain polymethacrylate polymers to, *inter alia,* control the swelling of inulin. The polymethacrylate polymers tested were Eudragit® RS; Eudragit® RL; 1:1 mixtures of Eudragit® RS and Eudragit® RL; Eudragit® FS; and 1:1 mixtures of Eudragit® RS and Eudragit® S.

US5422121 (Röhm GmbH) discloses an oral dosage form having a core containing at least one active ingredient enclosed within a shell material which comprises a polysaccharide that decomposes in the colon in admixture with a film-forming polymer. The ratio by weight of polysaccharide to film-forming polymer is from 1:2 to 5:1, preferably from 1:1 to 4:1. Premature diffusion of the active ingredient from the core can be suppressed using a gastric resistant isolating layer. The reference exemplifies *inter alia* tablets having an inner isolating layer of Eudragit® L30D with an outer layer comprising Eudragit® L30D and guar gum (Example 2) and tablets with a coating of Eudragit® S 100 and guar gum (Example 5).

WO96/36321A discloses an oral dosage form comprising a core containing bisacodyl, and an enteric polymer coating for the core, the coating comprising at least one inner coating layer and an outer coating layer. The or each inner coating layer is an enteric polymer that begins to dissolve in an aqueous medium at a pH from about 5 to about 6.3, and the outer coating layer is an enteric polymer that begins to dissolve in an aqueous medium at a pH from about 6.8 to about 7.2. The enteric polymer coating materials for the inner layer(s) are selected from the group consisting of cellulose acetate phthalate; cellulose acetate trimellitate; hydroxypropyl methylcellulose phthalate; hydroxypropyl methylcellulose acetate succinate; polyvinyl acetate phthalate; poly(methacrylic acid, methyl methacrylate) 1:1; poly(methacrylic acid, ethyl acrylate) 1:1; and compatible mixtures thereof.

WO2007/122374A discloses a colonic drug delivery formulation in which a mixture of a pH dependent film-forming polymeric material and a polysaccharide such as starch is used. Although it is known that this formulation shows delayed release followed by a relatively quick release of the drug, it would be desired if the drug release was even quicker in the colon.

WO2013/164315A discloses a colonic drug delivery formulation in which a mixture of a pH dependent film-forming polymeric material and a polysaccharide such as starch is used as an outer layer. An inner layer is used which is soluble in intestinal fluid or gastrointestinal fluid.

In accordance with a first aspect of the present invention, there is provided a delayed release drug formulation for oral administration to deliver a drug to the colon of a subject, said formulation comprising:
a core comprising a drug and optionally, an isolation layer; and
an outer coating for the core, the outer coating comprising a mixture of an enzymatically degradable polysaccharide which is susceptible to attack by colonic enzymes selected from the group consisting of starch, amylose, amylopectin, chitosan, chondroitin sulfate, cyclodextrin, dextran, pullulan, carrageenan, scleroglucan, chitin, curdulan, and levan; and a film-forming enteric polymer having a pH threshold at about pH 6 or above,
wherein the enzymatically degradable polysaccharide and the enteric polymer are present in the outer coating in a ratio of more than 60:40; and
wherein the outer coating is in direct contact with the surface of the uncoated core, or if present, the isolation layer.

It has been identified that delayed release drug formulations such as those disclosed in WO2013/164315A have a fast release profile in Krebs buffer (pH 7.4) under simulated fasting conditions across a broad range of ratios of polysaccharide to pH dependent material. However, the formation of formulations which have an optional isolation layer, an inner layer and a mixed material outer layer is potentially both complex and time consuming.

It has surprisingly been discovered that the delayed release drug formulations of the present invention advantageously also show a fast release profile in Krebs buffer (pH 7.4), which simulates the electrolyte composition of the distal small intestine, for compositions having a high polysaccharide content in the outer coating layer without the requirement of an inner layer. This is advantageous as it reduces both the time and cost of production compared with the comparative formulations with an additional layer.

### Enzymatically degradable polysaccharide

The polysaccharide is enzymatically degradable by one or more enzymes found in the colon of a subject. Such enzymes are produced by colonic bacteria and include amylases such as alpha-amylases, beta-amylases and iso-amlayses; amylopullunase, glucoamylase, alpha-glucosidase, maltogenic-amylase, glycosyltransferases and amylomaltase.

The person skilled in the art is capable of determining whether a particular polysaccharide is enzymatically degradable (*i.e.* susceptible to attack by colonic bacteria) using techniques comprising part of the common general knowledge. For example, a pre-determined amount of a given polysaccharide could be exposed to an assay containing an enzyme from a bacterium found in the colon and the change in weight of the material over time may be measured.

The enzymatically degradable polysaccharide is selected from the group consisting of starch; amylose; amylopectin; chitosan; chondroitin sulfate; cyclodextrin; dextran; pullulan; carrageenan; scleroglucan; chitin; curdulan; levan and hemicellulose such as xylan, glucuronoxylan, arabinoxylan, glucomannam, xyloglucan. Of these, starch; amylose; amylopectin; chitosan; chondroitin sulfate; cyclodextrin; and carrageenan are preferred. A particularly preferred polysaccharide is starch.

Starches are usually extracted from natural sources such as cereals; pulses; and tubers. Suitable starches for use in the present invention are typically food grade starches and include rice starch; wheat starch; corn (or maize) starch; pea starch; potato starch; sweet potato starch; tapioca starch; sorghum starch; sago starch; and arrow root starch. The use of maize starch is exemplified below.

Starch is typically a mixture of two different polysaccharides, namely amylose and amylopectin. Different starches may have different proportions of these two polysaccharides. Most natural (unmodified) maize starches have from about 20 wt % to about 30 wt % amylose with the remainder being at least substantially made up of amylopectin.

Suitable starches include "high amylose" and "low amylose" starches. High amylose starches are particularly preferred.

"High amylose" starches, are starches having at least 50 wt % amylose. Particularly suitable starches have from about 50 wt % to about 75 wt % amylose, preferably from about 50 wt % to about 70 wt %, more preferably from about 50 wt % to about 65 wt %, most preferably from about 50 wt % to about 60 wt %, e.g. about 55 wt %.

"Low amylose" starches are starches having less than 50 wt % amylose and at least 50 wt % amylopectin, e.g. up to 75 wt % amylopectin and even as much as up to 99 wt % amylopectin.

Starches suitable for use in the present invention typically have at least 0.1 wt %, e.g. at least 10 wt % or 15 wt %, preferably at least 35 wt %, amylose. Such starches have no more than 99.9 wt %, e.g. no more than 90 wt % or 85 wt %, preferably no more than 65 wt %, amylopectin. Such starches may have up to about 99 wt % amylose and no less than 1 wt % amylopectin.

Starches suitable for use in the present invention may have up to 100% amylopectin, more typically from about 0.1 wt % to about 99.9 wt % amylopectin. The starch may be, for instance, unmodified waxy corn starch. This typically comprises about 100% amylopectin.

Preferred starches have no more than 50 wt % amylopectin. Particularly suitable starches have from about 25 wt % to about 35 wt % amylopectin, e.g. about 30 wt % amylopectin.

The person skilled in the art is capable of determining the relative proportions of amylose and amylopectin in any given starch. For example, near-infrared (NIR) spectroscopy could be used to determine the amylose and amylopectin content of a starch using calibration curves obtained by NIR using laboratory-produced mixtures of known amounts of these two components. Further, starch could be hydrolysed to glucose using amyloglucosidase. A series of phosphorylation and oxidation reactions catalysed by enzymes result in the formation of reduced nicotinamide adenine dinucleotide phosphate (NADPH). The quantity of NADPH formed is stoichiometric with the original glucose content. Suitable test kits for this procedure are available (*e.g.,* R-Biopharm GmbH, Germany). Another method that could be used involves subjecting the coating to digestion by bacterial enzymes, *e.g.* α-amylase, to produce short chain fatty acids (SCFA) which can be quantified by gas-liquid chromatography using a capillary column.

Preferred starches have amylose in its glassy form although amylose in its amorphous form may also be used in conjunction with the present invention.

Preferred starches are "off-the-shelf" starches, *i.e.* starches which require no processing prior to use in the context of the present invention. Examples of particularly suitable "high amylose" starches include Eurylon® 6 (or VI) or Amylo N-400 (Roquette, Lestrem, France), or Amylogel 03003 (Cargill, Minneapolis, USA) all of which are examples of a maize starch having from about 50 wt % to about 75 wt % amylose.

### Film-forming enteric polymer

The film-forming enteric polymer is pH sensitive and has a pH threshold at about pH 6 or above. The "pH threshold" is the pH below which it is insoluble in aqueous media, and at or above which it is soluble in aqueous media. The pH of the surrounding medium therefore triggers dissolution of the enteric polymer, and none (or essentially none) of the enteric polymer dissolves below the pH threshold. Once the pH of the surrounding medium reaches (or exceeds) the pH threshold, the enteric polymer becomes soluble.

By "insoluble" we mean that 1 g of a polymeric material requires more than 10,000 ml of solvent or "surrounding medium" to dissolve at a given pH.

By "soluble" we mean that 1 g of a polymeric material requires less than 10,000 ml, preferably less than 5,000 ml, more preferably less than 1000 ml, even more preferably less than 100 ml or 10 ml of solvent or surrounding medium to dissolve at a given pH.

"Surrounding medium", preferably means the medium in the gastrointestinal tract, such as gastric juice or intestinal juice. Alternatively, the surrounding medium may be the *in vitro* equivalent of the medium in the gastrointestinal tract.

The normal pH of gastric juice is usually in the range of pH 1 to 3. The enteric polymer is insoluble below pH 6, and soluble at about pH 6 or above and, thus, is usually insoluble in gastric juice.

The pH of intestinal fluid may vary from one person to the next, but in healthy humans is generally from about pH 5 to 6 in the duodenum, from about 6 to 8 in the jejunum, from about 7 to 8 in the ileum, and from about 6 to 8 in the colon. The enteric polymer preferably has a pH threshold of about 6.5, *i.e.* is insoluble below pH 6.5 and soluble at about pH 6.5 or above, and more preferably has a pH threshold of about 7 or above, *i.e.* is insoluble below pH 7 and soluble at about pH 7 or above. The enteric polymer typically has a pH threshold of no more than about pH 8, *e.g.* no more than about pH 7.5 and preferably no more than about pH 7.2. Preferably, the enteric polymer has a pH threshold within the range of pH found in the colon.

The pH threshold at which a polymer becomes soluble may be determined by a simple titration technique which would be part of the common general knowledge to the person skilled in the art.

Suitable enteric polymers contain groups that are ionisable in aqueous media to form anions. Such polymers are known in the art as "anionic" polymers. Suitable anionic polymers include polycarboxylic acid polymers, *i.e.* polymers or co-polymers that contain a plurality of carboxylic acid functional groups that are ionisable in aqueous media such as intestinal fluid, to form carboxylate anions. As the pH of the surrounding medium increases, the proportion of carboxylic acid groups that become carboxylate anions increases, thereby increasing the solubility of the polymer.

The film-forming enteric polymer is typically a polymethacrylate polymer, a cellulose polymer or a polyvinyl-based polymer. Examples of suitable cellulose polymers include cellulose acetate phthalate (CAP); and hydroxypropylmethylcellulose acetate succinate (HPMC-AS).

Particularly suitable enteric polymers include a co-polymer of a (meth)acrylic acid and a (meth)acrylic acid C₁₋₄ alkyl ester, for example, a copolymer of methacrylic acid and methacrylic acid methyl ester. Such a polymer is known as a poly(methacrylic acid/methyl methacrylate) co-polymer. The ratio of carboxylic acid groups to methyl ester groups (the "acid:ester ratio") in these co-polymers determines the pH at which the co-polymer is soluble. The acid:ester ratio may be from about 2:1 to about 1:3, *e.g.* about 1:1 or, preferably, about 1:2. The molecular weight (MW) of preferred anionic co-polymers is usually from about 120,000 to 150,000 g/mol, preferably about 125,000 g/mol or about 135,000 g/mol.

Preferred anionic poly(methacrylic acid/methyl methacrylate) co-polymers have a molecular weight of about 125,000 g/mol. Suitable examples of such polymers have an acid:ester ratio of about 1:1 and a pH threshold of about pH 6, or have an acid:ester ratio of about 1:2 and a pH threshold of about pH 7.

A specific example of a suitable anionic poly(methacrylic acid/methyl methacrylate) co-polymer having a molecular weight of about 125,000 g/mol, an acid:ester ratio of about 1:1 and a pH threshold of about pH 6 is sold under the trade mark Eudragit® L. This polymer is available in the form of a powder (Eudragit® L 100), or as an organic solution (12.5%) (Eudragit® L 12.5).

Another specific example of a suitable anionic poly(methacrylic acidmethyl methacrylate) co-polymer is available in the form of an aqueous dispersion (30%) and is sold under the trade mark Eudragit® FS 30 D.

The Eudragit® co-polymers are manufactured and/or distributed by Evonik GmbH, Darmstadt, Germany.

Mixtures of film-forming enteric polymers may be used as appropriate. For example, the enteric polymer may actually be a blend of at least two different polymers having a pH threshold of about pH 6 or above. Preferably, the polymers in the blend are different polymethacrylate polymers. In embodiments where the enteric polymer is a blend of two different polymers having a pH threshold of about pH 6 or above, the polymers may be present in the blend in a polymer weight ratio from about 1:99 to about 99:1, *e.g.* from about 10:90 to about 90:10, or from 25:75 to about 75:25, or from about 40:60 to about 60:40, for example about 50:50.

An example of a suitable mixture would include a mixture, *e.g.* a 1:1 mixture, of Eudragit® L and Eudragit® S. A further example would include a blend, *e.g.* a 50:50 blend, of Eudragit® S and Eudragit® FS.

For the avoidance of doubt, the terms "mixture" and "blend" in the context of mixtures or blends of polymers forming the enteric polymer, are used herein interchangeably.

However, the use of a particular film-forming polymer material, *e.g.* a poly(methacrylic acid/methyl methacrylate) co-polymer, alone is preferred.

The use of Eudragit® S alone as the second polymeric material is particularly preferred.

### Outer coating layer

In a preferred embodiment, it has been found that a mixture of two suitable polymers at an appropriate ratio, applied as a film coating on to a core, at least minimises, and can substantially eliminate, drug release in the stomach and small intestine. Subsequent drug release in the colon is believed to occur by the combined active physiological triggers, *i.e.* by dissolution of the second material, particularly Eudragit® S, and digestion of the first material, e.g. starch or amylose.

Tthe proportion of the enzymatically degradable polysaccharide to the film-forming enteric polymer is at least 60:40. Typically, the proportion is less than 99:1. The proportion is preferably from about 65:25 to about 90:10, more preferably from about 70:30 to about 80:20, and most preferably abouy 75:25.

Optionally, conventional excipients such as those excipients selected from plasticisers for film formation (*e.g.* triethyl citrate) and anti-tack agents (*e.g.* glyceryl monostearate (GMS) or talc) and surfactants (*e.g.* polysorbate 80), may be included in amounts up to 30% by weight of the final composition of the outer coating preparation.

The thickness of the outer coating of the core is typically from about 10 µm to about 300 µm. The thickness of a specific coating will, however, depend on the composition of the coating and the size of the core. For example, coating thickness is directly proportional to the amount of polysaccharide in the coating.

In embodiments where the outer coating comprises high amylose starch and Eudragit® S at a ratio of about 75:25, the coating thickness may be from about 70 µm to about 300 µm, and preferably from about 150 µm to about 250 µm. The thickness (in µm) for a given coating composition is dependent on core size.

The outer coating typically has a coating amount of enteric polymer of from about 2 mg/cm² to about 10 mg/cm², *e.g.* from about 2 mg/cm² to about 8 mg/cm², or from about 3 mg/cm² to about 7 mg/cm², or from about 4 mg/cm² to about 6 mg/cm², or from about 5 mg/cm² to about 8 mg/cm², or from about 6 mg/cm² to about 8 mg/cm², or from about 7 mg/cm² to about 8 mg/cm², e.g. about 7.5 mg/cm², based on the dry weight of the enteric polymer. A typical core has a diameter of from about 5 x 10⁻⁴ m to about 25 mm.

By saying that the outer coating comprises a mixture of the polysaccharide and enteric polymer, it is intended to exclude the known multi-layer dosage form (disclosed for example in Milojevic *et al.* described above) in which an active core is coated first with an inner coating of amylose and then with an outer coating of Eudragit® L 100. In the context of the present invention, such a multi-layer dosage form does not comprise a mixture of starch and Eudragit® L 100.

The outer coating is preferably a single layer of a mixture of the polysaccharide and enteric polymer.

### Optional additonal layers

The outer coating layer of the invention is in direct contact with the surface of the core, that is the outer coating layer is in direct contact with the drug itself, or with a mixture of the drug and one or more excipients forming an uncoated core, or with the surface of an isolated core, *i.e.* a core coated with an isolation layer. Thus, the formulation of the present invention may have an additional isolation layer as part of the core, *i.e.* between the surface of the active core and the outer coating layer.

There may be formulations according to the present invention in which the composition of the core is incompatible with the outer coating. In such cases, it may be desirable to include an isolation layer to separate the core from the outer coating. For example, the present invention embraces embodiments in which the core contains a drug having acidic groups which may alter the functionality of an anionic polymer in the outer coating layer. An example of a drug having an acidic group would be 5-ASA. In such cases, it would typically be appropriate to include an isolation layer.

Any suitable isolation layer known to the skilled person can be used. In one preferred embodiment, the isolation layer comprises a film-forming non-ionic polymer. Suitable non-ionic polymers include methylcellulose (MC); hydroxypropyl cellulose (HPC); hydroxypropyl methylcellulose (HPMC, or hypromellose); poly(ethyleneoxide)-graft-polyvinylalcohol; polyvinylpyrollidone (PVP); polyethylene glycol (PEG); and polyvinylalcohol (PVA). Non-ionic cellulose based polymers (such as HPMC) are preferred, as is PVA. Mixtures of non-ionic polymers can also be used. A particularly preferred mixture is HPMC and PEG. The isolation layer can additionally comprise a plasticiser. Suitable plasticisers include but are not limited to polyethylene glycol, triethyl citrate, triacetin and acetyltriethyl citrate.

A final polishing layer may be applied to the outer coating as is known in the art.

### The core

The "core" is the solid body on which the outer coating is applied. The core may be any suitable dosage form, for example, a tablet, a pellet, a granule, a microparticle, a hard or soft capsule, or a microcapsule. In preferred embodiments, the core is a tablet or a capsule.

The core comprises the drug(s). The drug(s) may be contained within the body of the core, for example within the matrix of a tablet or pellet, or within the contents encapsulated within a capsule. Alternatively, the drug may be in a coating applied to the core, for example where the core is a bead of edible material such as sugar, *e.g.* where the core is in the form of a nonpareil bead or dragee.

The core may consist of the drug(s) alone, or more usually may consist of the drug(s) and at least one pharmacologically acceptable excipient. In this connection, the core is typically a tablet or pellet and consists of a mixture of the drug(s) with a filler or diluent material, *e.g.* lactose or cellulose material such as microcrystalline cellulose; a binder, *e.g.* polyvinylpyrrolidone (PVP) or hydroxypropyl methylcellulose (HPMC); a disintegrant, *e.g.* croscarmellose sodium (*e.g.* Ac-Di-Sol®) and sodium starch glycolate (*e.g.* Explotab®); and/or a lubricant, *e.g.* magnesium stearate and talc. The core may be a compressed granulate comprising at least some of these materials or others with the same functionality.

The core may be uncoated or, as indicated above, the core may itself comprise a coating such as an isolation layer on to which the outer coating layer is applied.

The minimum diameter of each core is typically at least about 10⁻⁴ m, usually at least about 5 x 10⁻⁴ m and, preferably, at least about 10⁻³ m. The maximum diameter is usually no more than 30 mm, typically no more than 25 mm and, preferably, no more than 20 mm. In preferred embodiments, the core has a diameter from about 0.2 mm to about 25 mm, and preferably from about 0.2 mm to about 4 mm (*e.g.* for pellets or mini-tablets) or from about 15 mm to about 25 mm (*e.g.* for certain tablets or capsules). The term "diameter" refers to the largest linear dimension through the core.

The formulation may comprise a plurality of coated cores in order to provide a single dose of the drug(s), particularly in embodiments in which the core is "small", *e.g.* having a diameter of less than 5 mm. Multiunit dosage forms comprising coated cores having a diameter of less than 3 mm may be preferred.

The present invention has application in a multi-phasic drug release formulation comprising at least two pluralities of coated cores, *e.g.* coated pellets, in the same dosage form, *e.g.* a capsule, in which the coated cores of one plurality are differentiated from the coated cores of the or each other plurality by the coating. The coatings may differ from one plurality to the next in terms of coating thickness or composition, *e.g.* the ratio and/or identity of components. Multi-phasic drug release formulations would be particularly suitable for suffers of Crohn's disease affecting different regions along the intestine.

Release from formulations according to the present invention is typically delayed until at least the distal ileum and, preferably, the colon. Release from certain formulations may also be sustained. However, in preferred formulations, release is pulsatile.

The time between initial exposure to conditions suitable for drug release and the start of drug release is known as the "lag time". The lag time depends on a number of factors including coating thickness and composition and may vary from one patient to the next. Formulations according to the present invention usually display a lag time in colonic conditions of at least 10 minutes. In most embodiments, the lag time is from about 10 minutes to about 4 hours, preferable from about 60 minutes to about 250 minutes.

A formulation is usually defined as gastric resistant if there is less than 10 wt % drug release in acidic media after 2 hours. Formulations according to the present invention typically display far less than 10 wt % drug release in acidic media and may be considered to be gastric resistant. The formulations usually display less than 1 wt % drug release in acidic media and, typically, display substantially no drug release in acidic media. When starch is combined with an acrylate film-forming material to form the outer layer of the coating for the core, typically less than 5% drug release occurs over 5 hours in conditions simulating the stomach and small intestine.

By way of an example, in embodiments in which a tablet core is coated with a coating comprising a high amylose starch and Eudragit® S (75:25) with a coating amount of enteric polymer of 5.0 mg polymer/cm², based on the dry weight of enteric polymer, the time between initial release and complete release in Kreb's buffer may be less than about 4 hours, preferably less than about 3 hours.

In a preferred embodiment, the core is a pellet having a diameter of about 1 mm. In another preferred embodiment, the core is a tablet having a diameter of about 8 mm. In both cases, the coating is preferably a 75:25 mixture of high amylose starch, *e.g.* Eurylon® 6 (Amylo N-400), and an acrylic polymer, *e.g.* Eudragit® S. In both preferred embodiments, the core is coated to achieve a coating amount of enteric polymer of about 5.0 mg polymer/cm², based on the dry weight of enteric polymer.

The formulation of the present invention can comprise any size core. In some embodiments, the drug can be present in the core of the formulation in an amount of from about 50 mg to about 1650 mg, or from about 100 mg to about 1550 mg, or from about 150 mg to about 1500 mg, or from about 200 mg to about 1450 mg, or from about 250 mg to about 1400 mg, or from about 300 mg to about 1350 mg, or from about 350 mg to about 1300 mg, or from about 400 mg to about 1250 mg, or from about 450 mg to about 1200 mg, or from about 500 mg to about 1150 mg, or from about 550 mg to about 1100 mg, or from about 600 mg to about 1050 mg, or from about 650 mg to about 1000 mg, or from about 700 mg to about 950 mg, of from about 800 mg to about 1600 mg, or from about 850 mg to about 1600 mg, or from about 900 mg to about 1500 mg, or from about 950 mg to about 1400 mg or from about 1000 to about 1300 mg, or from about 1150 about 1200 mg. Preferably, the drug is present in the core amount selected from about 400 mg, about 800 mg, about 1200 mg, about 1500 mg, or about 1600 mg.

### Different aspects

According to a second aspect of the present invention, there is provided a formulation according to the first aspect for use in a method of medical treatment of the human or animal body by therapy.

The core comprises at least one drug. The formulation of the present invention is usually used to administer a single drug as the sole therapeutically active component. However, more than one drug may be administered in a single formulation.

The formulation of the present invention is designed to administer a wide range of drugs. Suitable drugs include those drugs which are known for intestinal administration using known delayed release oral formulations. The present invention may be used to administer drugs having a local or a systemic effect.

The formulation of the present invention has particular application in the intestinal administration of a drug comprising at least one acidic group such as a carboxylic acid group. Such drugs may be acidic drugs or zwitterionic drugs. An example of such a drug is 5-aminosalicylic acid (5-ASA, otherwise known as or mesalamine or mesalazine).

The identity of the drug(s) in the formulation obviously depends on the condition to be treated. In this connection, the formulation has particular application in the treatment of IBD (including Crohn's disease and ulcerative colitis); IBS; constipation; diarrhoea; infection; and carcinoma, particularly colon or colorectal cancer.

For the treatment or prevention of IBD, the formulation may comprise at least one drug selected from the group consisting of anti-inflammatory agents (*e.g.* 5-ASA, 4-ASA, sulphasalazine and balsalazide); non-steroidal anti-inflammatory agents (*e.g.* ibuprofen and diclofenac); steroids (*e.g.* prednisolone; budesonide or fluticasone); immunosuppressants (*e.g.* azathioprine; cyclosporin; and methotrexate); antibiotics; and biological agents including peptides, proteins, antibodies and antibody fragments. Suitable examples of biological agents include alkaline phosphatase and anti-TNF antibodies such as infliximab, adalimumab, certulizumab pegol, golimumab and ustekinumab.

For the treatment or prevention of cancer, the formulation may comprise at least one antineoplastic agent. Suitable antineoplastic agents include fluorouracil; methotrexate; dactinomycin; bleomycin; etoposide; taxol; vincristine; doxorubicin; cisplatin; daunorubicin; VP-16; raltitrexed; oxaliplatin; and pharmacologically acceptable derivatives and salts thereof. For the prevention of colon cancer or colorectal cancer, primarily in patients suffering from colitis, the formulation may comprise the anti-inflammatory agent, 5-ASA.

For the treatment or prevention of IBS, constipation, diarrhoea or infection, the formulation may comprise at least one active agent suitable for the treatment or prevention of these conditions.

Pharmacologically acceptable derivatives and/or salts of the drugs may also be used in the formulation. An example of a suitable salt of prednisolone is methyl prednisolone sodium succinate. A further example is fluticasone propionate.

The present invention has particular application in either the treatment of IBD (particularly, ulcerative colitis) or the prevention of colon cancer or colorectal cancer (primarily in colitis patients), both using 5-ASA. It also has application as a portal of entry of drugs into the systemic circulation via the colon. This is particularly advantageous for peptide and protein drugs which are unstable in the upper gastrointestinal tract. The present invention may also be utilised for the purpose of chronotherapy.

In a third aspect of the invention, there is provided a method of targeting a drug to the colon comprising administering to a patient a formulation as defined above.

In a fourth aspect of the invention, there is provided the use of a formulation as defined above in the manufacture of a medicament for the treatment or prevention of IBD (particularly ulcerative colitis); IBS; constipation; diarrhoea; infection; and cancer.

There is also provided the use of at least one drug selected from anti-inflammatory agents and steroids in the manufacture of a medicament comprising a formulation as defined above for use in the treatment of IBD. In addition, there is also provided the use of at least one antineoplastic agent in the manufacture of a medicament comprising a formulation as defined above for use in the treatment of carcinoma. Further, there is also provided use of 5-ASA in the manufacture of a medicament comprising a formulation as defined above for use in the prevention of colon cancer or colorectal cancer.

According to a fifth aspect of the present invention, there is provided a method of medical treatment or prevention of IBD or carcinoma comprises administering to a patient a therapeutic amount of a formulation as defined above.

The formulation will typically comprise a therapeutically effective amount of the or each drug which may be from about 0.01 wt % to about 99 wt %, based on the total weight of the formulation. The actual dosage would be determined by the skilled person using his common general knowledge. However, by way of example, "low" dose formulations typically comprise no more than about 20 wt % of the drug, and preferably comprise from about 1 wt % to about 10 wt %, e.g. about 5 wt %, of the drug. "High" dose formulations typically comprise at least 40 wt % of the drug, and preferably from about 45 wt % to about 85 wt %, e.g. about 50 wt % or about 80 wt %.

### Method

According to a sixth aspect of the present invention, there is provided a method of producing a delayed release formulation according to the first aspect.

The method comprises the steps of:
forming a core comprising a drug and, optionally, an isolation layer; and
coating the core directly with an outer coating preparation comprising an enzymatically degradable polysaccharide which is susceptible to attack by colonic enzymes selected from the group consisting of starch, amylose, amylopectin, chitosan, chondroitin sulfate, cyclodextrin, dextran, pullulan, carrageenan, scleroglucan, chitin, curdulan, and levan; and a film-forming enteric polymer having a pH threshold at about pH 6 or above in a solvent, to form an outer coated core,
wherein the enzymatically degradable polysaccharide and the enteric polymer are present in the outer coating layer in a ratio of more than 60:40.

Preferred polysaccharides and enteric polymers are as detailed above. The core is preferably spray coated with said outer coating preparation.

In a preferred embodiment, the method comprises initially coating the core with an isolation layer coating preparation, typically comprising a non-ionic cellulose-based film-forming polymer in a solvent, to form an isolated core for coating with the outer coating preparation.

In embodiments in which the core is formed from a compressed granulate, the method preferably comprises:
dry mixing the drug(s) with at least one excipient to form a dry mixture;
granulating at least a portion of said dry mixture to form a granulate;
compressing at least a portion of said granulate to form said core; and
spray coating said core with said outer coating preparation to form said delayed release drug formulation.

The granulation step may be carried out using either a wet granulation or a dry granulation process. Preferably granulation is carried out using a wet granulation process.

In alternative embodiments, the core can be formed by a direct compression process or by a layering process.

A fluidised bed spray coating machine or a perforated pan coater can be used to coat the core(s) with the outer coating preparation to form the particle(s) of the formulation.

In preferred embodiments, the method comprises:
forming an aqueous dispersion or solution comprising said polysaccharide;
forming an alcoholic or aqueous solution comprising said enteric polymer; and
adding, preferably drop-wise, at least a portion of said aqueous dispersion of said polysaccharide to at least a portion of said alcoholic or aqueous solution of said enteric polymer to form said outer coating preparation.

In embodiments in which the polysaccharide is starch, it is preferred that the starch is dispersed in at least one alcohol, preferably a C₁ to C₆ alcohol, e.g. methanol; ethanol; propan-1-ol; propan-2-ol; butan-1-ol; butan-2-ol; and mixtures thereof, particularly butan-1-ol alone, and then water is usually added subsequently with good agitation. The resulting aqueous dispersion is usually heated to reflux (*i.e.* "cooked") and then cooled with stirring overnight. The purpose of the alcohol(s) is to solvate the starch ready to form the aqueous dispersion. Alternatively, the polysaccharide can be dispersed directly in water.

In addition, where starch is used as the polysaccharide, the alcohol assists with stabilizing leached amylose during the cooking process. Specifically, it is believed that amylose forms V-complexes with butan-1-ol, which decrease the rate of crystallization upon cooling. The reassociation of amylose into double helices is known as retrogradation and is thought to decrease the digestibility of the starch.

The enteric polymer is typically dissolved in at least one solvent, for instance water or an organic solvent. The organic solvent may be an alcohol, *e.g.* methanol; ethanol; propan-2-ol; methyl glycol; butyl glycol; acetone; methyl glycol acetate; and mixtures thereof such as acetone and isopropyl alcohol (*e.g.* in a ratio of about 4:6). The enteric polymer is preferably dissolved in ethanol (preferably from 85 to 98%) under high speed stirring.

The outer coating preparation is preferably formed by adding an appropriate quantity of the aqueous dispersion to the alcoholic solution, drop-wise under fast stirring. The further excipient(s) such as a plasticiser (*e.g.* triethyl citrate) and/or a lubricant (*e.g.* glyceryl monostearate) is usually added to the preparation while stirring.

### EXAMPLES

A number of preferred embodiments of the present invention will now be described with reference to the drawings, in which:-
FIG. 1 is a graph comparing drug release as a function of time from coated 5-ASA tablets according to Example 1, Example 2, Example 3 and Comparative Example 1, when exposed to 0.1M HCI for 2 hours and then Krebs buffer (pH 7.4) for 8 hours;
FIG. 2 is a graph comparing drug release as a function of time from coated 5-ASA tablets according to Example 2, when exposed to 0.1N HCl for 2 hours and then Sørensen's buffer (pH 6.8), (a) with α-amylase and (b) without α-amylase.

### Materials

Eudragit® S 100, was purchased from Evonik GmbH, Darmstadt, Germany. Maize starch (Eurylon® 6) was purchased from Roquette, Lestrem, France. Polysorbate 80 (Tween® 80), butan-1-ol, triethylcitrate (TEC), ethanol 95%, potassium phosphate monobasic (KH₂PO₄), sodium diphosphate dibasic dihydrate (Na₂HPO₄•2H₂O), and sodium hydroxide were all purchased from Sigma-Aldrich, Buchs, Switzerland. Hydroxypropyl methylcellulose (HPMC, Pharmacoat® 603) was purchased from Shin-Etsu. Polyethylene glycol 6000 (PEG 6000) was purchased from Fluka. Glyceryl monostearate (GMS) was purchased from Cognis. Iron oxide red and iron oxide yellow (Sicovit) were purchased from BASF.

### Tablet cores

Tablet cores containing 5-ASA were provided. For Example 1, cores of 800 mg were used. For Example 2, cores of 1200 mg were used. For Example 3 and Comparative Example 1, cores of 1600 mg were used.

The tablet cores for Example 1-3 and Comparative Example 1 were coated with an isolation layer comprising HPMC and polyethylene glycol 6000 (PEG 6000).

### Preparation of coated tablet cores

EXAMPLES 1, 2 and 3 (HPMC isolation layer/outer coating of a 25:75 mixture of Eudragit® S 100 and high amylose starch)

### Isolation layer

The isolation layer was applied from an aqueous mixture of HPMC and 20% PEG 6000 (based on dry polymer weight) in the following amounts:

**Table 1**

| **Component** | **mg/cm²** |
|---|---|
| HPMC | 3 |
| PEG 6000 | 0.6 |

The HPMC was dissolved in water under magnetic stirring and then the PEG 6000 was added to form an isolation layer coating preparation. The isolation layer coating preparation was sprayed onto the 5-ASA cores using a perforated pan coater until the coating amount of HPMC reached 3 mg polymer/cm² to form isolation layer coated cores. The spray coating parameters were as follows:

**Table 2**

| | |
|---|---|
| Drum speed (rpm) | 10 |
| Nozzle diameter (mm) | 1.0 |
| Spray rate (g/min) | 3 |
| Spray pressure (bar) | 0.7 |
| Pattern pressure (bar) | 1.0 |
| Air flow (m³/h) | 40 |
| Inlet air temperature (°C) | 58 - 65 |
| Outlet air temperature (°C) | 40 - 42 |

### Outer coating

The isolation layer coated tablet cores were coated with an outer coating formed of 25% Eudragit® S 100 and 75% high amylose starch.

The outer coating was applied from a mixture of an aqueous starch dispersion and an ethanolic Eudragit® S 100 solution in the following amounts (based on Eudragit® S 100 dry polymer weight):

**Table 3**

| **Component** | **mg/cm²** |
|---|---|
| Starch | 17.15 |
| GMS | 1.0 |
| Polysorbate 80 | 0.4 |
| Iron Oxide yellow | 0.11 |
| Iron Oxide red | 0.66 |
| Eudragit® S 100 | 5 |
| Triethyl citrate | 4 |

The aqueous starch dispersion was prepared by dispersing high amylose maize starch, (Eurylon® 6 also known as Amylo N-400) into butan-1-ol, followed by water, under magnetic stirring. The resulting dispersion was heated to boiling and then cooled under stirring overnight.

The Eudragit® S 100 solution was prepared by dispersing Eudragit® S 100 in 96% ethanol under high speed stirring.

The aqueous starch dispersion was added dropwise to the Eudragit® S 100 solution under stirring to obtain a ratio of Eudragit® S 100:starch of 25:75. The mixture was stirred for 1 hour and triethyl citrate and a GMS emulsion (previously prepared with Polysorbate 80) were added and mixed for further 30 minutes. A suspension of iron oxide red and iron oxide yellow was added and the mixture was stirred for a further 10 minutes.

The GMS emulsion was prepared at a concentration of 5% w/w. Polysorbate 80 (Tween, 40% based on GMS weight) was dissolved in distilled water followed by dispersion of the GMS. The dispersion was heated at 75 °C for 15 minutes under strong magnetic stirring in order to form an emulsion. The emulsion was cooled at room temperature under stirring.

The pigment suspension was formed by suspending red and yellow iron oxide pigments in 96% ethanol for 10 minutes under homogenization.

The final outer coating preparation was sprayed onto the isolation layer coated cores using a perforated pan coater until the coating amount of Eudragit® S 100 reached 5 mg polymer/cm². The spray coating parameters were as follows:

**Table 4**

| | |
|---|---|
| Drum speed (rpm) | 12 - 14 |
| Nozzle diameter (mm) | 1.0 |
| Spray rate (g/min) | 4.0 - 5.1 |
| Spray pressure (bar) | 0.4 |
| Pattern pressure (bar) | 0.5 |
| Air flow (m³/h) | 40 |
| Inlet air temperature (°C) | 57 - 60 |
| Outlet air temperature (°C) | 40 - 41 |

COMPARATIVE EXAMPLE 1 (HPMC isolation layer/inner layer of partially neutralized Eudragit® S 100/outer coating of a 25:75 mixture of Eudragit® S 100 and high amylose starch)

### Isolation layer

The isolation layer was prepared and applied according to Examples 1, 2 and 3.

### Inner layer

The inner layer was applied from an aqueous preparation of methacrylic acid-methyl methacrylate copolymer, ratio 1:2 (Eudragit® S 100), where the pH was adjusted to pH 8. The composition of the inner layer also included 70% triethyl citrate (based on dry polymer weight), 10% GMS (based on dry polymer weight), 40% Polysorbate 80 (based on GMS weight), and 1% KH₂PO₄ buffer agent (based on dry polymer weight).

The inner layer was applied by spray coating in the in the following amounts (based on Eudragit® S 100 dry polymer weight):

**Table 5**

| **Component** | **mg/cm²** |
|---|---|
| Eudragit® S 100 | 5 |
| KH₂PO₄ | 0.05 |
| Triethyl citrate | 3.5 |
| Glyceryl monostearate | 0.5 |
| Polysorbate 80 | 0.2 |
| 1M NaOH | As required to reach pH 8 |

KH₂PO₄ was dissolved in distilled water, followed by dispersion of the Eudragit® S 100. Thereafter, triethyl citrate and a GMS emulsion (previously prepared with Polysorbate 80) were added and mixed for further 30 minutes. The pH was then adjusted using 1M NaOH until pH 8 was obtained.

The final inner layer coating preparation was sprayed onto the isolation layer coated cores using a perforated pan coater until the amount of Eudragit® S 100 reached 5 mg polymer/cm². The spray coating parameters were as follows:

**Table 6**

| | |
|---|---|
| Drum speed (rpm) | 10-12 |
| Nozzle diameter (mm) | 1.0 |
| Spray rate (g/min) | 4 |
| Spray pressure (bar) | 0.6 |
| Pattern pressure (bar) | 0.8 |
| Air flow (m³/h) | 40 |
| Inlet air temperature (°C) | 60 - 65 |
| Outlet air temperature (°C) | 39 - 40 |

### Outer coating

The outer coating was applied from a mixture of an aqueous starch dispersion and an aqueous Eudragit® S 100 solution. The outer coating preparation was formed as in Examples 1, 2 and 3 and sprayed onto the inner layer coated cores until the amount of Eudragit® S 100 reached 5 mg polymer/cm². The spray coating conditions were as used in Examples 1, 2 and 3.

### Drug release test #1 - Simulated fasted state then dissolution in Krebs Buffer at pH 7.4

*In vitro* dissolution studies were performed on a USP type II apparatus using a paddle speed of 50 rpm and a media temperature of 37 ± 0.5 °C. To simulate the "fasted" state, tablets were first tested in 0.1 M HCl for 2 hours followed by 8 hours in Krebs buffer (pH 7.4).

### Drug release test #2 - Simulated fasted state then dissolution in Sorenson buffer at pH 6.8 (without α-amylase)

*In vitro* dissolution studies were performed on a USP type II apparatus using a paddle speed of 50 rpm and a media temperature of 37 ± 0.5 °C. The coated tablets were tested in Sørenson buffer at pH 6.8 (35.4 mM KH₂PO₄ + 35.6 mM NaH₂PO₄).

To simulate the "fasted" state, tablets were first tested in 0.1 M HCl for 2 hours followed by 10 hours in Sørenson buffer at pH 6.8.

### Drug release test #3 - Simulated fasted state then dissolution in Sorenson buffer at pH 6.8 (effect of α-amylase)

*In vitro* dissolution studies were performed on a USP type II apparatus using a paddle speed of 50 rpm and a media temperature of 37 ± 0.5 °C. The coated tablets were tested in Sørenson buffer at pH 6.8 (35.4 mM KH₂PO₄ + 35.6 mM NaH₂PO₄) containing 50 U (units)/ml α-amylase derived from *B*. *licheniformis.*

To simulate the "fasted" state, tablets were first tested in 0.1 M HCI for 2 hours followed by 10 hours in Sørenson buffer at pH 6.8.

### Results

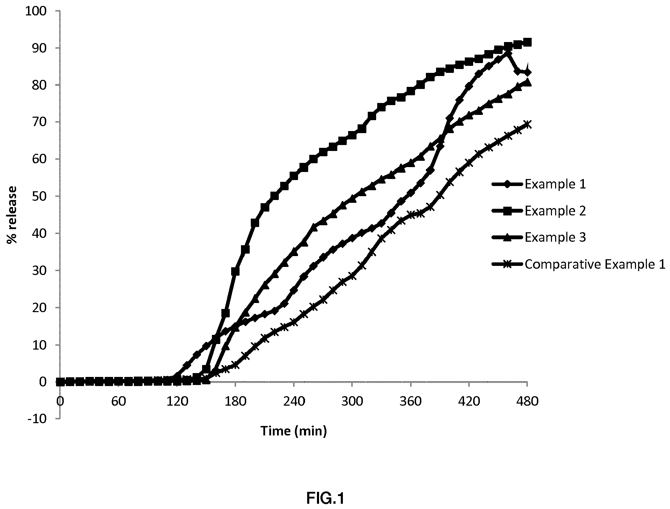

The results presented in FIG. 1 demonstrate that the formulations according to Examples 1, 2 and 3 showed similar dissolution properties to those according to Comparative Example 1, independent of the core size used in each example. Specifically, upon exposure to pH 7.4 all tablets tested demonstrated a similar drug release profile, with lag times in the region of 130-160 minutes (drug release test #1, FIG. 1). This demonstrates that the absence of an alkaline inner layer in Examples 1, 2 and 3 does not have a negative effect on the release characteristics of the tablets.

It is surprising that the absence of the inner layer in Examples 1, 2 and 3 does not have an impact on the drug release profile when the outer coating has a high amount of polysaccharide since the inner layer was developed to provide drug release acceleration above the pH threshold of the enteric polymer. This is observed with lower ratios of polysaccharide to enteric polymer.

### Enzymatic digestion

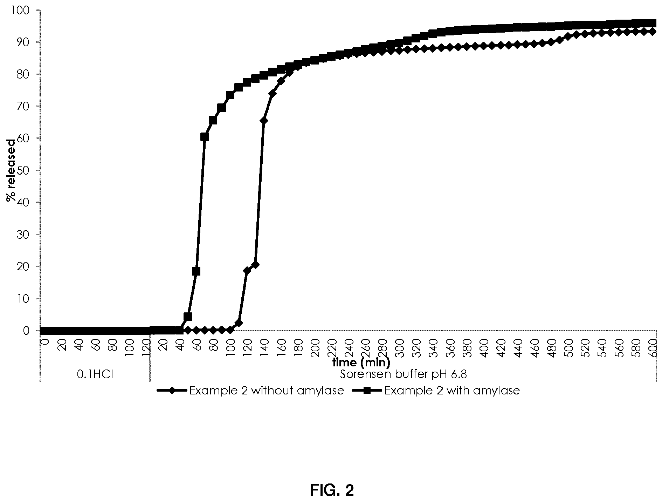

In aqueous solution at pH 6.8, enzymatic triggered release is observed for tablets of Example 2 and 3 (drug release tests #2 and #3). Specifically, an earlier initial release of 5-ASA is observed when α-amylase is present in the buffer solution at pH 6.8. Interestingly, drug release from the single layer coated tablets of Examples 2 and 3 is significantly faster than the double layer coated tablets of Comparative Example 1 at pH 6.8, both with and without α-amylase (Table 7, FIG. 2).

**Table 7**

| | Lagtime in Sorensen buffer pH 6.8 with amylase (min) | Lagtime in Sorensen buffer pH 6.8 without amylase (min) |
|---|---|---|
| Example 2 | 60 | 120 |
| Example 3 | 90 | 120 |
| Comparative Example 1 | > 600 | 530 |

It will be appreciated that the invention is not restricted to the details described above with reference to the preferred embodiments but that numerous modifications and variations can be made without departing from the scope of the invention as defined by the following claims.

## Claims

1. A delayed release drug formulation for oral administration to deliver a drug to the colon of a subject, said formulation comprising:
a core comprising a drug and optionally, an isolation layer; and
an outer coating for the core, the outer coating comprising a mixture of an enzymatically degradable polysaccharide which is susceptible to attack by colonic enzymes selected from the group consisting of starch, amylose, amylopectin, chitosan, chondroitin sulfate, cyclodextrin, dextran, pullulan, carrageenan, scleroglucan, chitin, curdulan, and levan; and a film-forming enteric polymer having a pH threshold at about pH 6 or above,
wherein the enzymatically degradable polysaccharide and the enteric polymer are present in the outer coating in a ratio of more than 60:40; and
wherein the outer coating is in direct contact with the surface of the uncoated core or, if present, the isolation layer.

2. A delayed release drug formulation as claimed in Claim 1, wherein the enzymatically degradable polysaccharide and the enteric polymer are present in the outer coating in a ratio of about 65:35 to about 90:10, more preferably from about 70:30 to about 80:20, most preferably about 75:25.

3. A delayed release drug formulation as claimed in Claim 1 or Claim 2, wherein the enteric polymer is present in the outer coating in an amount from about 4 mg/cm² to about 6 mg/cm², preferably about 5 mg/cm², based on the dry weight of the enteric polymer.

4. A delayed release drug formulation as claimed in any of the preceding claims, wherein the core comprises an isolation layer.

5. A delayed release drug formulation as claimed in any of the preceding claims, wherein the isolation layer comprises a film-forming non-ionic polymer.

6. A delayed release drug formulation as claimed in Claim 5, wherein the non-ionic polymer is a non-ionic cellulose-based polymer.

7. A method of producing a delayed release drug formulation for oral administration to deliver a drug to the colon as claimed in Claim 1, said method comprising:
forming a core comprising a drug and, optionally, an isolation layer; and
coating the core directly with an outer coating preparation comprising an enzymatically degradable polysaccharide which is susceptible to attack by colonic enzymes selected from the group consisting of starch, amylose, amylopectin, chitosan, chondroitin sulfate, cyclodextrin, dextran, pullulan, carrageenan, scleroglucan, chitin, curdulan, and levan; and a film-forming enteric polymer having a pH threshold at about pH 6 or above in a solvent, to form an outer coated core,
wherein the enzymatically degradable polysaccharide and the enteric polymer are present in the outer layer in a ratio of more than 60:40.

8. A method as claimed in Claim 7 comprising initially coating the core with an isolation layer coating preparation comprising a film-forming non-ionic polymer in a solvent to form an isolated core for coating with the outer coating preparation.

9. A method as claimed in Claim 8, wherein the non-ionic polymer is a non-ionic cellulose-based polymer.

10. A method as claimed in any of Claims 7 to 9, wherein the enzymatically degradable polysaccharide and the enteric polymer are present in the outer coating preparation in a ratio of about 65:35 to about 90:10, preferably from about 70:30 to about 80:20, and more preferably about 75:25.

11. A method as claimed in any of Claims 7 to 10, wherein the core is coated with the outer coating preparation until the enteric polymer is coated on to the core in an amount from about 4 mg/cm² to about 6 mg/cm², preferably about 5 mg/cm², based on the dry weight of the enteric material.
